# EUROPEAN PATENT APPLICATION

(11) **EP 1 260 216 A1**
(43) Date of publication of application: **27.11.2002**
(21) Application number: 02007778.0
(22) Date of filing: 06.04.2002
(51) Int. Cl.: A61K 9/24, A61K 9/00, A61K 9/48

(54) **Multi-layered pharmaceutical composition for both intraoral and oral administration**

(30) Priority: 15.05.2001 US 858016
(71) Applicant: Peirce Management, LLC, Wellesley, MA 02481 (US)
(72) Inventor: Hirsh, Jane C., Wellesley, MA 02481 (US); Midha, Kamal K., Hamilton HM 12, Bermuda (US); Hirsh, Mark, Wellesley, MA 2481 (US); Lo, Whe-Yong, Canton, MA 02021 (US)
(74) Representative: COHAUSZ DAWIDOWICZ HANNIG & PARTNER

(57) **Abstract**

New pharmaceutical composition in unit dosage form 1 are disclosed for both intraoral and oral administration to a mammalian patient, said unit dosage form configured to be placed intraorally of said patient, which comprises:
(a) as a first portion 3 , at least one discrete outer layer comprising a therapeutically effective amount of at least one pharmaceutically active ingredient capable of intraoral administration; and
(b) as a second portion 4 located within said first portion 3, a therapeutically effective amount of at least one pharmaceutically active ingredient capable of oral administration and which is releasable and orally ingestible by the patient after the outer layer has disintegrated or has dissolved intraorally.

## Description

### FIELD OF THE INVENTION

This invention relates to pharmaceutical compositions for both intraoral (i.e. sublingual or buccal or vestibular mucosa or gingival) and oral (swallowing whole of after chewing) administration. More particularly this invention relates to compositions containing at least one pharmaceutically active ingredient capable of intraoral administration and at least one pharmaceutically active ingredient capable of oral administration once the intraorally administered active ingredient has been substantially released. The invention further relates to a method of administering a pharmaceutical composition for both intraoral and oral administration to a patient or other mammalian subject.

### BACKGROUND OF THE INVENTION

Intraoral administration of medicaments has been carried out according to the prior art. See U.S. Patent 4,229,447 to PORTER. This patent discloses the intraoral administration of benzodiazepines including diazepam, lorazepam, oxazapam, temazapam, and chlordiazepoxide. According to Porter it is known in the art to administer benzodiazapines either orally or parenterally (i.e. by injection), especially intramuscularly or subcutaneously. Administering benzodiazepines by injection enables rapid attainment of effective plasma concentrations, that is more rapid than the plasma concentrations obtained following oral administration. One advantage of the intraoral administration as opposed to parenteral administration is that there is no injection site where pain and inflammation may develop. Another such advantage is that intraoral administration does not require sterilization of the preparations and the hypodermic syringes. Furthermore in many situations self-administration of a medicament by parenteral means is not possible for a patient.

In U.S. Patent 5,739,136 to Ellinwood Jr. et al the medicament selected for intraoral administration is one that upon oral administration is metabolized into an unwanted or aversive metabolite that is increasingly formed during gastrointestinal tract absorption and subsequent portal vein entry into the liver. Examples of such medicaments include not only the benzodiazepines especially a trifluoro-benzodiazepine such as quazepam, but also other medicaments where it is advantageous to avoid first pass metabolism such as the antianxiety/anticonvulsant/antihypnotic agents propoxyphene, nefazodone, trazodone, chlorimipramine, bupropion and combinations thereof. Unlike Porter, Ellinwood Jr. et al is not only concerned with rapidly attaining of effective plasma concentrations of the intraorally administered medicament, but is especially concerned that the medicament include a drug where first pass metabolism is to be avoided. The intraoral administration of the antianxiety/anticonvulsant/antihypnotic agents decreases the metabolism to the undesired metabolites.

U.S. Patents 5,053,032, 5,248,310, 5,573,776, and 5,776,493 each assigned to ALZA Corporation disclose delivery systems for deliverying a beneficial drug to the oral cavity of a patient. The delivery systems include a signalling device that informs the patient when the beneficial drug has been entirely delivered. Nowhere in these prior art references, however, is there a disclosure of a pharmaceutical composition that effectively administers a therapeutically effective amount of pharmaceutically active ingredients by both intraoral means to obtain a rapid onset of desired plasma concentration and by oral means to obtain a more sustaining as well as complementary therapeutic effect. Nowhere in these references is there any disclosure of such a signalling system that is used not only to inform the patient when the pharmaceutical composition for intraoral administration has been depleted, but to also inform the patient that it is time to either chew and swallow or swallow whole the remainder of the composition to orally administer a second pharmaceutically active ingredient.

### OBJECTS OF THE INVENTION

It is an object of the invention to provide pharmaceutical compositions containing at least one pharmaceutically active ingredient capable of intraoral administration and at least one pharmaceutically active ingredient capable of oral administration and which is releasable and orally ingestible by the patient or other mammalian subject after the pharmaceutically active ingredient capable of intraoral administration has disintegrated or has dissolved intraorally. It is a further object of the invention to provide a process for the preparation of a pharmaceutical composition containing at least one pharmaceutically active ingredient capable of intraoral administration and at least one pharmaceutically active ingredient capable of oral administration and which is releasable and orally ingestible by the patient after the pharmaceutically active ingredient capable of intraoral administration has disintegrated or has dissolved intraorally.

It is a further object of the invention to provide a method of treatment employing the new pharmaceutical compositions for both intraoral and oral administration that is easily administrable to a patient or other mammalian subject so as to maximize therapeutic effectiveness and minimize side effects.

### SUMMARY OF THE INVENTION

We have found that these objects can be achieved if an active ingredient for intraoral administration is in a tablet or capsule suitable for intraoral administration to a patient. According to one feature the present invention is directed to a method of treating a patient by intraorally administering to the patient. a therapeutically effective amount of a pharmaceutically active ingredient capable of intraoral administration. The tablet or capsule also contains a therapeutically effective amount of a pharmaceutically active ingredient capable of oral administration which is releasable and orally ingestible by the patient after the pharmaceutically active ingredient capable of intraoral administration has disintegrated or has dissolved intraorally.

According to a preferred feature of the present invention, the pharmaceutical composition in unit dosage form is in the form of a tablet or capsule for both intraoral and oral administration configured to be placed intraorally and comprises:
(a) as a first portion, at least one discrete outer layer comprising a therapeutically effective amount of at least one pharmaceutically active ingredient capable of intraoral administration; and
(b) as a second portion located within said first portion, or in a layer of a multi-layer tablet, a therapeutically effective amount of at least one pharmaceutically active ingredient capable of oral administration and which is releasable and orally ingestible by the patient after the outer coating layer or outer layer of a multi-layer tablet has disintegrated or has dissolved intraorally.

Such tablets or capsules are administered preferably once or twice per day, either a.m. or p.m. and contain for intraoral dosing 1 mcg - 50 mg, preferably 5 mcg to 40 mg and more preferably 10 mcg to 30 mg pharmaceutically active ingredient capable of intraoral administration. The pharmaceutical compositions further contain 5 mcg to 2 Gm, preferably 5 mg to 1000 mg and more preferably 10 mg to 500 mg of the pharmaceutically active ingredient capable of oral administration.

The present compositions are best suited where disease symptoms are such that both rapid relief of some of these symptoms are required as well as a continuation in controlling similar or other symptoms. Thus the outer layer preferably contains a medicament suitable for intraoral administration in a dosage of 0.1 to 30 mg and the molecular weight of the pharmaceutically active compound generally does not exceed 350 daltons. Generally the pharmaceutically active compounds used for intraoral administration would have a low bioavailability upon oral administration as opposed to intravenous administration. These pharmaceutically active compounds undergo presystemic metabolism/clearance (i.e. first pass metabolism). These active ingredients are metabolized during their first passage through the gut wall and liver and thereby a significant fraction of the dose administered does not enter the bloodstream, and does not reach the receptors to exert activity. At times avoidance of pre-systemic metabolism results in avoiding the metabolism of the active drug into unwanted or aversive metabolites. Other times it allows lower doses of the active drug to provide desired activity as compared to oral dosing. The pharmaceutically active ingredients capable of intraoral administration and the pharmaceutically active ingredients capable of oral administration may include analgesics, antihistamines, antidiarrheals, anxiolytics, hypnotics, stimulants, cardiovascular drugs, pulmonary drugs, anti-hypertensives, antiemetics, anti-inflammatory drugs, renal drugs, steroids, drugs for neurological disorders, anticonvulsant drugs, drugs for treating endocrine disorders, drugs for sexual dysfunction, drugs for promoting immunology, drugs for treating osteoarthritis, drugs for treating glaucoma, drugs for treating allergic rhinitis, drugs for treating anemias and other hematological disorders, drugs for treating infectious diseases, drugs for the treatment and symptoms of cancer, drugs for insomnia, and antidiabetic drugs.

The pharmaceutically active ingredient capable of intraoral administration must be capable of sublingual or buccal absorption through the mucous membranes of the mouth at its therapeutically effective level. Such an active ingredient may have a first pass metabolism that may be avoided by the intraoral administration and preferably has a rapid onset through the intraoral administration.

The pharmaceutically active ingredient capable of oral administration and orally administered may be the same pharmaceutically active ingredient that is capable of intraoral administration and intraorally administered. The orally administered pharmaceutically active ingredient may be immediately released, may be continuously released or may be delayed released after a period of 0.5 to 12 hours. The core component of a dosage form is formulated as a chewable or swallowable composition for immediate or modified release. The pharmaceutically active ingredient capable of oral administration and orally administered may be a different pharmaceutically active ingredient from the intraorally administered pharmaceutically active ingredient. In this case the orally administered pharmaceutically active ingredient is concomitantly administered to achieve the therapeutic benefits of the combined pharmaceutically active ingredients such as the relief of different symptoms of the same disease or to synergistically increase the total therapeutic effect of the individual pharmaceutically active ingredients.

To summarize the features which are unique for such a delivery system include:
- 1.: Pharmacological complimentarity.
- 2.: Reduced dosage of the active ingredient.
- 3.: Reduced side effects.
- 4.: Versatility which provides immediate as well as sustained and prolonged therapeutic benefit.
- 5.: A.M./ P.M. administration with clear advantages in terms of desired effect.
- 6.: Improved compliance.

Preferred examples of the pharmaceutically active ingredients capable of intraoral administration that are known in the art, based on their physicochemical, pharmacokinetic and pharmacodynamic properties are many. These active ingredients are not limited to those which include:

Buprenorphine, Butorphanol, Fenoprofen, Flurbiprofen, Diflunisal, Naproxen, Ibuprofen, Potassium Diclofenac, Fentanyl, Sodium Diclofenac, Parecoxib, Valdecoxib, Ketorolac, Tramadol, Aceclofenac, Acetaminophen, Acetanilide, Alphaprodine, Codeine, Dihydrocodeine, Dihydromorphine, Hydrocodone, Hydromorphone, Indoprofen, Ketoprofen, Ketorolac, Levorphanol, Loxoprofen, Meperidine, Methadone, Morphine, Naloxone, Pentazocine, Norlevorphanol, Oxycodone, Oxymorphone, Phenazocine, Propoxyphene, Tramadol, Zomepirac, Zolpidem, Zaleplon, Bupropion, Lorazepam, Buspirone, Ipsapirone, Gepirone, Triazolam, Nitrazepam, Estazolam, Diazepam, Oxazepam, Acetazolamide, Amiloride, Butazolamide, Clofenamide, Disulfamide, Ethacrynic Acid, Ethiazide, Ethoxzolamide, Etozolin, Fenquizone, Furosemide, Hydrochlorothiazide, Quinethazone, Torsemide, Triamterene, Cyproheptadine, Acravistine, Azatadine Maleate, Brompheniramine, Dextromethorphan, Carinoxamine Maleate, Chlorpheniramine, Descarboxyethyloratadine, Fexofenadine, Loratadine, Clemastine, Pseudoephedrine, Dexbrompheniramine, Diphenhydramine, Norastemizole, Doxylamine, Ketotifen, Promethazine, Tripelennamine, Plaunotol, Rosaprostol, Rotraxate, Spizofurone, Teprenone, Troxipide, Zolimidine, Temelastine, Naratriptan, Dizatriptan, Frovatriptan, Zolmitriptan, Sumatriptan, Almotriptan, Rizatriptan, Propanolol, Atenolol, Amitryptyline, Nortriptyline, Doxepin, Verapamil, Amlodipine, Ergotamine Tartrate, Caffeine, Dihydroergotamine, Bupropion, Methylphenidate, Captopril, Clonidine, Betaxolol, Bisoprolol, Nitroglycerin, Isorbide Mononitrate, Isosorbide Dinitrate, Bufuralol, Furosemide, Guanabenz, Guanfacine, Hydralazine, Labetalol, Metoprolol, Nadolol, Nifedipine, Oxprenolol, Pindolol, Sotalol, Timolol, Bunitrolol, Indecaimide, Nadoxolol, Penbutolol, Tocainide, Hydrochlorothiazide, Furosemide, Granisetron, Odansetron, Prochlorperazine, Metoclopramide, Clonazepam, Vigabatrin, Sildenafil, Alprostadil, Fentanyl, Sufentanil, Lofentanil, Carfentanil, Alfentanil, Pentacozine, Nalbuphine, Ketamine, Droperidol, Haloperidol, Valium, Midazolam, Lorazepam, Etomidate, Pentathol, Methohexitol, Deprivan, Propanidid, Minoxalone.

To those skilled in the art, it is well known that physicochemical properties can be influenced by other inert agents which include penetration enhancers, pH modifiers and other taste masking agents.

Examples of the pharmaceutically active ingredients capable of oral administration include:

Rofecoxib, Fenoprofen, Flurbiprofen, Diflunisal, Naproxen, Ibuprofen, Indomethacin, Etodolac, Sulindac, Fentanyl, Sodium Diclofenac, Celecoxib, Parecoxib, Valdecoxib, Ketorolac, Tramadol, Aceclofenac, Acetaminophen, Acetanilide, Acetylsalcylsalicylic Acid, Alphaprodine, Aspirin, Bermoprofen, Naprosyn, Codeine, Dihydrocodeine, Dihydromorphine, Hydrocodone, Hydromorphone, Indoprofen, Ketoprofen, Ketorolac, Levorphanol, Loxoprofen, Mefenamic Acid, Meperidine, Methadone, Morphine, Nabumetone, Pentazocine, Norlevorphanol, Normethadone (N-desmethyl-methadone), Normorphine (N-desmethyl-morphine), Oxycodone, Oxymorphone, Phenazocine, Propoxyphene, Salsalate, Suprofen, Tramadol, Zomepirac, Zolpidem, Zaleplon, Zopiclone, Hydroxyzine, Trazodone, Citalopram, Lorazepam, Buspirone, Ipsapirone, Gepirone, Triazolam, Nitrazepam, Estazolam, Diazepam, Oxazepam, Acetazolamide, Amanozine, Amiloride, Benzylhydrochlorothiazide, Bumetanide, Buthiazide, Chlorothiazide, Clofenamide, Clopamide, Clorexolone, Cyclothiazide, Disulfamide, Ethacrynic Acid, Ethiazide, Ethoxzolamide, Etozolin, Fenquizone, Furosemide, Hydracarbazine, Hydrochlorothiazide, Hydroflumethiazide, Indapamide, Mefruside, Methazolamide, Methyclothiazide, Metolazone, Pamabrom, Torsemide, Trimterene, Xipamide, Spironolactone, Cyproheptadine, Meclizine, Brompheniramine, Dextromethorphan, Citirizine, Chlorpheniramine, Descarboxyethyloratidine, Fexofenadine, Loratadine, Pseudoephedrine, Dexbrompheniramine, Diphenhydramine, Norastemizole, Doxylamine, Ketotifen, Promethazine, Tripelennamine, Cimedtidine, Famotidine, Lansoprazole, Nizatidine, Omeprazole, Pantoprazole, Pirenzepine, Plaunotol, Ranitidine, Rebamipide, Rioprostil, Roxatidine Acetate, Telenzepine, Troxipide, Zolmidine, O-Desmethyl astemizole, Acrivastine, Temelastine, Naratriptan, Dizatriptan, Zolmitriptan, Sumatriptan, Almotriptan, Eletriptan, Rizatriptan, Propanolol, Atenolol, Amitryptyline, Nortriptyline, Doxepin, Verapamil, Amlodipine, Ergotamine tartrate, Caffeine, Dihydroergotamine, Amoxapine, Bupropion, Citalopram, Clomipramine, Desipramine, Doxepin, Fluoxetine, Fluvoxamine, Paroxetine, Trazodone, Vanlafaxine, d-amphetamine, Captopril, Enalapril, Lisinopril, Quinapril, Acebutolol, Benzapril, Nitroglycerin, Isorbide Mononitrate, Isosorbide Dinitrate, Bupranolol, Corvedilol, Condesortan, Diltiazem, Doxazosin, Felodipine, Fosinopril, Furosemide, Guanabenz, Guranfacine, Hydralazine, Indopamide, Irbesarton, Labetalol, Isradepine, Linopril, Losarton, Metolazone, Metoprolol, Mibefradil, Moexipril, Nadolol, Nicardipine, Nifedipine, Nisoldipine, Oxprenolol, Pindolol, Prazosin, Propranolol, Quinapril, Rampipril, Sotalol, Telmisartan, Temocapril, Terazosin, Trandolapril, Valsartan, Verapamil, Amiodarone, Aprindine, Bufetolol, Bunitrolol, Encainide, Flecainide, Indecaimide, Mexiletine, Nadoxolol, Penbutolol, Practolol, Propafenone, Quinidine Sulphate, Tocainide, Bumetanide, Loperamide, Granistron, Dolasetron Mesylate, Ondansetron, Dexamethasone, Prochlorperazine, Metoclopramide, Gabapenten, Carbamazepine, Clonazepam, Lamotrigine, Phenytoin, Tigabine, Vigabatrin, Sildenafil, Papaverine.

Pharmaceutically acceptable acid addition salts of the pharmaceutically active ingredient capable of intraoral administration or of the pharmaceutically active ingredient capable of oral administration and capable of forming pharmaceutically acceptable acid addition salts within the scope of this invention include the salts of any pharmaceutically acceptable inorganic acid or organic acid. Such pharmaceutically acceptable inorganic acids may include hydrochloric, hydrobromic, sulfuric, nitric, or phosphoric acid. Pharmaceutically acceptable organic acids may include acetic, propanoic, 2-hydroxyacetic, 2-hydropxypropanoic, 2-oxopropanoic, propanedioic, butanedioic, methanesulfonic, ethanesulfonic, benzenesulfonic, hydroxybenzoic, malic, tartaric, maleic, fumaric, adipic, citric, ascorbic or cinnamic acid.

In the tablets or capsules for intraoral and oral administration the outer coating contains a pharmaceutically active ingredient capable of intraoral administration and the core contains a pharmaceutically active ingredient capable of oral administration. The resulting pharmaceutical composition may be therapeutically effective over a duration of several hours, especially where the pharmaceutically active ingredient capable of oral administration contained in the core is in sustained-release or delayed-release form.

Not only may the core of the pharmaceutical composition contain an additional active ingredient capable of oral administration, but furthermore the coating may also contain an additional pharmaceutically active ingredient capable of intraoral administration. In fact the core and the coating may both contain additional pharmaceutically active agents.

The outer coating for intraoral administration may include one or more pharmaceutically acceptable excipients such as lactose, sucrose, dextrose, mannitol, starch, polyvinylpyrrolidone, microcrystalline, talc and magnesium stearate for compression coat. Other inert ingredients that may be included in the outer coating include polymers such as polyethylene glycol, methylcellulose, hydroxypropyl methylcellulose, an emulsifier or dispersing agent such as Polysorbate 80, a glidant such as talc and a coloring agent.

The core may include in addition to the pharmaceutically active ingredient capable of oral administration and the optional compounds capable of oral administration, a pharmaceutically acceptable inert carrier such as lactose, sucrose, dextrose or mannitol as well as a binding or disintegrating agent such as microcrystalline cellulose. The core may also include a glidant such as colloidal silica and may include a tablet lubricant such as magnesium stearate. The core may also include a viscosity increasing agent such as various grades of hydroxypropyl methyl cellulose as well as povidone. The ingredients in the core are blended to form a blend and the blend is then granulated with a binder such as povidone in water. The granules are then dried and milled to the desired particle size and blended with a glidant agent such as colloidal silica and a lubricant such as magnesium stearate.

The pharmaceutical compositions comprising both an outer coating and a core include a 'signalling system' such as flavor particles interspersed between the outer coating suitable for intraoral administration and the core wherein the flavor of the flavor particles is capable of detection by the patient only once the outer coating has dissolved. The flavor may be any flavor detectable by the patient within the mouth or under the tongue. The flavor may include fruit flavors such as grape, cherry, orange, lemon, strawberry, or raspberry or spice flavors such as cinnamon or cloves. The flavoring may also include tart agents such as tartaric acid or citric acid and is not detectable by the patient who has the pharmaceutical composition within his mouth until the outer coating has substantially been dissolved or has disintegrated. Once the patient detects and responds to the signalling system, the patient is instructed to swallow the core thereby beginning the process of orally administering the therapeutically effective amount of the medicament found in the core. Other signalling systems include color change of the dosage form, gas liberation or effervescence, texture (surface change from outer to inner layer), sensation (hot or cold from menthol, camphor, ginger, capsicum), and local anesthesia.

Once the core is formed by blending, granulating, milling and compressing, a signalling system is applied to the surface of the core. The core is then ready for application of the coating containing the pharmaceutically active ingredient capable of intraoral administration.

In addition to the layer of flavor particles that may be interspersed between the core and the coating on the tablet, there may be additional flavor particles but different from those for signalling, dispersed throughout the coating in order to make the intraoral administration more pleasant for the patient. The flavor particles in the coating may be chosen from among the same flavors as in the flavor particles interspersed between the core and the coating. However, the particular flavor of the flavor particles in the coating cannot be the same flavor as the flavor particles interspersed between the core and the coating; otherwise the patient will not know when the coating has disintegrated and the time has come to swallow the core. The primary purpose of adding one or more flavoring agents is to inform the patient when it is time to swallow or chew the second portion (inner part) of the composition containing the pharmaceutically active ingredient capable of oral administration and providing the patient with a more pleasant taste for the pharmaceutically active ingredient in the first portion administered intraorally or for the pharmaceutically active ingredient in the second portion administered orally is secondary.

Alternative methods for a signalling system for the patient to detect when the pharmaceutically active ingredient capable of intraoral administration has been substantially released by the dissolved or disintegrated first portion of the composition are: color change of the dosage form, sound popping, gas liberation or effervescence, texture (surface change from outer to inner layer), sensation (hot or cold from e.g. menthol, camphor, ginger, capsicum), and local anesthetic (e.g. benzocaine, eugenol, lidocaine, spilanthol). When the patient detects the signalling system, the patient is informed to either chew or swallow whole the remainder of the composition. Preferably the signalling system informs the patient that the intraoral drug administration has been substantially completed 2 minutes to 2 hours after the pharmaceutical composition has been taken.

The pharmaceutically acceptable signalling system may be located in a separate layer between the first portion and second portion of the composition, within the first portion of the composition or within the second portion of the composition and that is detectable by the patient upon substantial release of the pharmaceutically active ingredient capable of intraoral administration during intraoral administration thereby informing the patient that it is time to orally ingest the remaining second part of the composition containing the pharmaceutically active ingredient capable of oral administration.

To determine whether it is better to include the signalling system in a separate layer between the first and second parts of the composition or within either the first or the second part of the composition, the properties of the pharmaceutically active ingredients for intraoral and for oral administration must be considered, such as the compatibility of the pharmaceutically active ingredients with the signalling system, the need to mask an undesirable taste for one or both of the pharmaceutically active ingredients, and the size of the dose for one or both of the pharmaceutically active ingredients.

According to another feature of the present invention the coating is then compressed as an outer layer around the core.

The invention further comprises a method of administering a pharmaceutical composition in unit dosage form as a tablet or capsule for both intraoral and oral administration to a patient, which comprises:
(a) as a first portion, a discrete outer layer comprising a therapeutically effective amount of at least one pharmaceutically active ingredient capable of intraoral administration; and
(b) as a second portion located within said first portion, a therapeutically effective amount of at least one pharmaceutically active ingredient capable of oral administration and which is releasable and orally ingestible by the patient after the outer layer has disintegrated or has dissolved intraorally, which comprises the steps of:
   (i) placing the pharmaceutical composition within the mouth of said patient;
   (ii) retaining the pharmaceutical composition within the mouth of the patient until the first portion of the pharmaceutical composition containing the pharmaceutically active ingredient capable of intraoral administration has dissolved or has disintegrated thereby substantially releasing the pharmaceutically active ingredient capable of sublingual administration; and
   (iii) following step (ii) swallowing whole or chewing and swallowing the second portion of the pharmaceutical composition.

Preferably the pharmaceutical composition further comprises a pharmaceutically acceptable signaling system located between the first portion and second portion of the composition and which following step (ii) further comprises the step of detecting the signaling system to indicate to the patient substantial release of the pharmaceutically active ingredient capable of intraoral administration during intraoral administration in step (ii) thereby informing the patient that it is time to orally ingest the remaining second part of the composition containing the pharmaceutically active ingredient capable of oral administration according to step (iii). According to one feature of the invention the first portion of the compositions which contains the pharmaceutically active ingredient capable of intraoral administration disintegrates and/or dissolves rapidly, within 10 minutes, as soon as the composition comes into contact with the patient's saliva. The second portion of the compositions which contains the pharmaceutically active ingredient capable of oral administration remains intact and does not dissolve or disintegrate while the pharmaceutically active ingredient capable of intraoral administration is intraorally administered.

Where it is desired to allow a rapid sublingual and mucosal absorption of the pharmaceutically active ingredient capable of intraoral administration, the outer layer in the first portion containing the pharmaceutically active ingredient capable of intraoral administration will dissolve or disintegrate substantially immediately.

Once the outer layer in the first portion has dissolved or disintegrated, the second portion of the composition is either swallowed whole or chewed by the patient to release the pharmaceutically active ingredient capable of oral administration.

The present invention also relates to a process for the preparation of a pharmaceutical composition in unit dosage form as a tablet or capsule for both intraoral and oral administration to a patient, said pharmaceutical composition placed intraorally in said patient, which comprises:
(a) as a first portion, at least one discrete outer layer comprising a therapeutically effective amount of at least one pharmaceutically active ingredient capable of intraoral administration; and
(b) as a second portion located within said first portion, a therapeutically effective amount of at least one pharmaceutically active ingredient capable of oral administration and which is releasable and orally ingestible by the patient after the at least one outer layer has disintegrated or has dissolved intraorally which comprises the steps of:
   (i) providing the second portion as an inner tablet core or as at least one layer of a multi-layer tablet core or as an uncoated capsule; and
   (ii) applying the first portion as an outer coating on the inner core or capsule surface or as one or more outer layers of the multi-layer tablet.

In the case of a tablet the first portion is a coating layer which is applied to an inner core or one or two outer layers of a multi-layer tablet. The second portion of the tablet is an inner core or a layer of a multi-layer tablet. In the case of a capsule the outer layer is a coating layer applied on the outer surface of the capsule. The second portion of the capsule is the capsule itself and the contents of the capsule.

The tablet outer coating is preferably a film coat that is applied to the inner core or is a compression coating that is compressed around the inner core. The film coat comprises at least one pharmaceutically active ingredient capable of intraoral administration and at least one pharmaceutically acceptable one pharmaceutically active ingredient capable of intraoral administration; and
(b) as a second portion located within said first portion, a therapeutically effective amount of at least one pharmaceutically active ingredient capable of oral administration and which is releasable and orally ingestible by the patient after the at least one outer layer has disintegrated or has dissolved intraorally which comprises the steps of:
   (i) providing the second portion as an inner tablet core or as at least one layer of a multi-layer tablet core or as an uncoated capsule; and
   (ii) applying the first portion as an outer coating on the inner core or capsule surface or as one or more outer layers of the multi-layer tablet.

In the case of a tablet the first portion is a coating layer which is applied to an inner core or one or two outer layers of a multi-layer tablet. The second portion of the tablet is an inner core or a layer of a multi-layer tablet. In the case of a capsule the outer layer is a coating layer applied on the outer surface of the capsule. The second portion of the capsule is the capsule itself and the contents of the capsule.

The tablet outer coating is preferably a film coat that is applied to the inner core or is a compression coating that is compressed around the inner core. The film coat comprises at least one pharmaceutically active ingredient capable of intraoral administration and at least one pharmaceutically acceptable coating polymer selected from the group consisting of cellulose, hydroxypropyl methylcellulose, methyl cellulose, polyvinylpyrrolidone, and polyethylene glycol, a pharmaceutically acceptable plasticizer, a pharmaceutically acceptable glidant and a pharmaceutically acceptable colorant. The compression coat comprises at least one pharmaceutically active ingredient capable of intraoral administration and is formulated with pharmaceutically acceptable excipients for intraoral administration such as lactose, sugar, sorbitol, mannitol, microcrystalline cellulose, starch, gelatin, sodium starch glycolate, polyvinylpyrrolidone, polyethylene glycol, and magnesium stearate. Alternatively the compression coat may comprise at least one pharmaceutically active ingredient capable of intraoral administration and at least one pharmaceutically acceptable excipient for intraoral administration such as an alkali carbonate or bicarbonate capable of rendering effervescence when contacted with water.

The tablet may be a multi-layer tablet where the outer layer comprises the pharmaceutically active compound for intraoral administration and at least one pharmaceutically acceptable excipient for intraoral administration such as lactose, sugar, sorbitol, mannitol, microcrystalline cellulose, starch, sodium starch glycolate, polyvinylpyrrolidone, polyethylene glycol, and magnesium stearate. Alternatively the outer layer in a multi-layer tablet comprising at least one pharmaceutically active ingredient capable of intraoral administration and at least one pharmaceutically acceptable effervescent agent such as an alkali carbonate or bicarbonate capable of rendering effervescence when contacted with water.

In any of the compositions disclosed hereinabove the outer coating or layer may contain a pharmaceutically acceptable flavoring agent.

The inner core in one feature of the present invention is an immediate drug release tablet. Preferably the immediate drug release tablet comprising the inner core comprises the pharmaceutically active ingredient capable of oral administration and a conventional pharmaceutically acceptable excipient for oral administration such as lactose, calcium sulfate, calcium phosphate dibasic, sugar, microcrystalline cellulose, methylcellulose, starch, sodium starch glycolate, polyvinylpyrrolidone, polyethylene glycol and magnesium stearate. The inner core may comprise one or more compressed layers (e.g. bilayer, trilayer) and where the inner core comprises multiple layers, one or more of the layers may contain the pharmaceutically active ingredient capable of oral administration in immediate release form.

In another feature of the present invention the inner core is a sustained drug release tablet which provides sustained release of the pharmaceutically active ingredient capable of oral administration. Preferably the sustained release effect lasts for 0.5 to 24 hours. The sustained drug release core comprises the pharmaceutically active ingredient capable of oral administration and a conventional pharmaceutically acceptable excipient for oral administration in sustained drug release form such as methylcellulose, hydroxypropyl methylcellulose, ethyl cellulose, cellulose acetate phthalate, acacia, wax and glycerol monostearate, acrylic acid polymers and copolymers, methacrylic acid, methyl acrylate, ethyl acrylate, methyl methacrylate, ethyl methacrylate, and a conventional pharmaceutical excipients for oral administration such as lactose, calcium sulfate, calcium phosphate dibasic, sugar, microcrystalline cellulose, methylcellulose, starch, sodium starch glycolate, polyvinylpyrrolidone, polyethylene glycol and magnesium stearate.

The inner core may comprise one or more layers and where the inner core comprises multiple layers, one or more of the layers may contain the pharmaceutically active ingredient capable of oral administration in sustained release form.

In addition to pharmaceutical compositions where the inner core of a tablet contains the pharmaceutically active ingredient capable of oral administration in immediate release form or sustained (extended) release form, also within the scope of the invention are compositions where the inner core is coated with a delayed release coating prior to coating with the outer coating to delay the release of the pharmaceutically active ingredient capable of oral administration. Preferably the delay in release lasts 0.5 to 12 hours. The delayed release coating is formulated with one or more pharmaceutically acceptable polymers selected from the group consisting of methylcellulose, hydroxypropyl cellulose, hydroxyethyl cellulose, hydroxymethyl cellulose, hydroxypropyl methylcellulose, cellulose acetate succinate, ethylcellulose, cellulose acetate phthalate, cellulose acetate trimellitate, carboxymethyl cellulose sodium, acrylic acid polymers and copolymers, methacrylic acid, methyl acrylate, ethyl acrylate, methyl methacrylate, ethyl methacrylate, polyvinylpyrrolidone, vinyl acetate, vinyl acetate phthalate, azo compounds, pectin, amylose, shellac, zein, and guar gum, in combination with a pharmaceutically acceptable plasticizer.

Also within the scope of the invention are tablet cores containing a pharmaceutically active ingredient for oral administration that contains an enteric coating so that the pharmaceutically active ingredient is not released until reaching the small intestines or even the large intestines (e.g. colon). The inner core containing the pharmaceutically active ingredient capable of oral administration may be swallowed whole by the patient or may be in chewable form. In the case of an inner core with multiple layers, one or more of the layers containing the pharmaceutically active ingredient capable of oral administration may be in chewable form. The inner core in chewable form or the inner core with multiple layers in chewable form comprises the pharmaceutically active ingredient capable of oral administration and a pharmaceutically acceptable excipient for chewable tablets selected from the group consisting of lactose, sorbitol, mannitol, sugar, starch, citric acid, and magnesium stearate, optionally with a flavoring agent.

As mentioned hereinabove capsules as well as tablets are a feature of the present invention. Just as the tablet outer coating may be a film coat, so the outer coating of the capsule may also be a film coat that is applied to the outer surface of the capsule. As in the film coat over the tablets the film coat over the capsules comprises the same pharmaceutically active ingredients suitable for intraoral administration, the same pharmaceutically acceptable coating polymers, the same plasticizers, the same glidants and the same colorants. The capsule outer coating may also include a pharmaceutically acceptable flavoring agent.

The second part (inner part) of the dosage form, the uncoated capsule, according to the present invention may comprise a pharmaceutically active ingredient capable of oral administration and in a form available for immediate release once the outer coating of the capsule has dissolved or has disintegrated intraorally, and is formulated with pharmaceutically acceptable excipients selected from the group consisting of lactose, starch, calcium phosphate, calcium sulfate, microcrystalline cellulose, talc and magnesium stearate.

Alternatively the second part (inner part) of the dosage form, the uncoated capsule, according to the present invention may comprise a pharmaceutically active ingredient capable of oral administration and in a form available for sustained (extended) release once the outer coating of the capsule has dissolved or has disintegrated intraorally. The sustained release effect preferably lasts for 0.5 to 24 hours. The second part of the dosage form is formulated with conventional pharmaceutically acceptable excipients for oral administration in sustained drug release form such as methylcellulose, hydroxypropyl methylcellulose, ethyl cellulose, cellulose acetate phthalate, acacia, wax and glycerol monostearate, acrylic acid polymers and copolymers, methacrylic acid, methyl acrylate, ethyl acrylate, methyl methacrylate, ethyl methacrylate, and one or more conventional pharmaceutical excipients for oral administration such as lactose, calcium sulfate, calcium phosphate dibasic, sugar, microcrystalline cellulose, methylcellulose, starch, sodium starch glycolate, polyvinylpyrrolidone, polyethylene glycol and magnesium stearate. The inner part of the dosage form, the capsule, may also be coated with a delayed release coating prior to coating with the outer coating to delay the release of the pharmaceutically active ingredient capable of oral administration. Preferably the delay in release lasts 0.5 to 12 hours. The delayed release coating is formulated with one or more pharmaceutically acceptable polymers selected from the group consisting of methylcellulose, hydroxypropyl cellulose, hydroxyethyl cellulose, hydroxymethyl cellulose, hydroxypropyl methylcellulose, cellulose acetate succinate, ethylcellulose, cellulose acetate phthalate, cellulose acetate trimellitate, carboxymethyl cellulose sodium, acrylic acid polymers and copolymers, methacrylic acid, methyl acrylate, ethyl acrylate, methyl methacrylate, ethyl methacrylate, polyvinylpyrrolidone, vinyl acetate, vinyl acetate phthalate, azo compounds, pectin, amylose, shellac, zein, and guar gum, in combination with a pharmaceutically acceptable plasticizer. Also within the scope of the invention are capsule cores containing a pharmaceutically active ingredient for oral administration that contains an enteric coating so that the pharmaceutically active ingredient is not released until reaching the small intestines or even the large intestines (e.g. colon).

The signalling agent may be coated on the inner core of the pharmaceutical compositions including the inner core of the composition in tablet form as well as immediately over the shell of the capsule containing the pharmaceutically active ingredient capable of oral administration when the composition is in capsule form. In both cases the first portion of the pharmaceutical composition containing the pharmaceutically active ingredient capable of intraoral administration overlays the coating containing the flavoring agent.

Alternatively particles of the flavoring agent may be coated with a sustained-release polymer such as methylcellulose, hydroxypropyl methylcellulose, ethyl cellulose, cellulose acetate phthalate, acacia, wax and glycerol and the particles embedded in the outer layer (first portion) of the respective compositions. In this way immediate release of the flavoring agent is avoided and detection of the flavoring agent by the patient before the outer coating containing the pharmaceutically active ingredient capable of intraoral administration has dissolved or has disintegrated is avoided.

In some situations a different flavoring agent may be coated on or included in the first portion (outer part) of the pharmaceutical composition from the flavoring agent that is coated on or included in the second portion (inner part) of the composition. The unique features of the delivery system of the invention include pharmacological complementarily, reduced side effects and versatility which provide immediate as well as sustained and prolonged therapeutic benefits along with improved patient compliance. For example a sleepless, anxious patient may receive medications which are complementary to control anxiety and at the same time receive a hypnotic agent to provide sleep. There is a pharmacological therapeutic complementarily where two agents control two different symptoms of a disease. Furthermore there is an advantage of having intraoral administration for rapid onset of a desired therapeutic effect subsequently maintained with oral dosing or subsequently maintained for sustained action with a modified oral active agent in the core.

### BRIEF DESCRIPTION OF THE DRAWINGS

Figure 1 is a cross sectional view of an embodiment of a dosage form of this invention wherein the dosage form is a tablet, and
Figure 2 is a cross sectional view of an embodiment of a dosage form of this invention wherein the dosage form is a capsule.

### SPECIFIC DESCRIPTION OF THE DRAWINGS

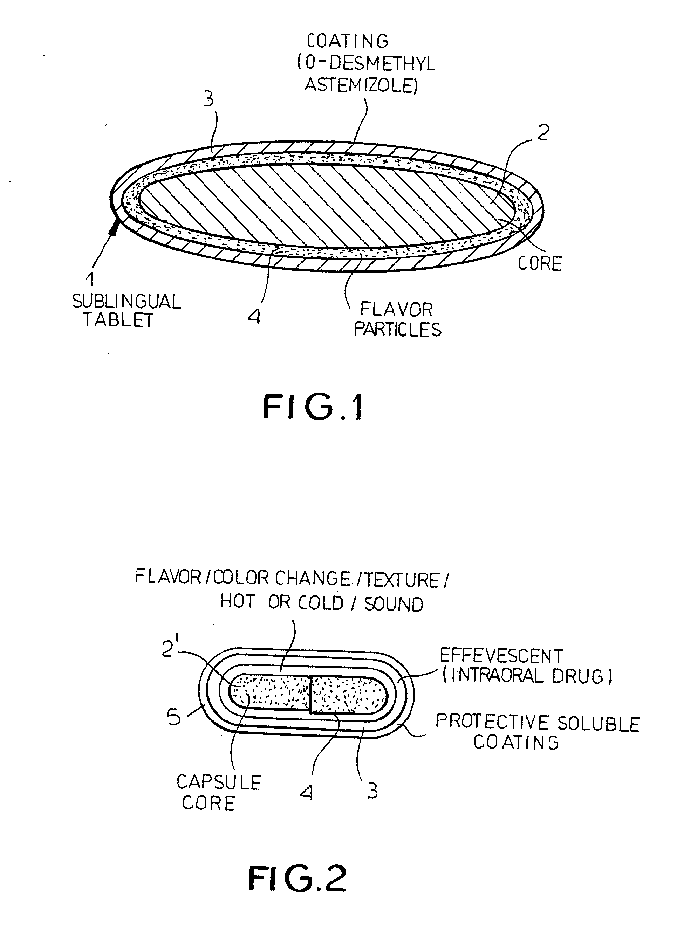

Figure 1 is a perspective drawing cut in section and which shows a tablet suitable for intraoral and oral administration. The tablet comprises an outer coating 1, which contains the intraoral portion 2, the flavoring signalling layer 3, delayed release coating, and 4, a core for oral administration. The outer coating 1 includes a therapeutically effective amount of a pharmaceutically active ingredient capable of intraoral administration which upon intraoral administration to the patient disintegrate and dissolves within said 10 minutes for intraoral administration. The inner core 4 contains a pharmaceutically active ingredient capable of oral administration. The signalling system 3 is coated over the core or delayed release coated core. This layer of flavor particles serves to inform the patient who has taken the tablet intraorally that the coating containing the pharmaceutically active ingredient capable of intraoral administration has essentially disintegrated in the patient's mouth and now all that remains is the core. The patient is instructed to swallow or chew the core and thus the core is administered just as any conventional oral medication once the outer coating has disintegrated.

Figure 2 shows an embodiment in which the core 4, is a capsule for oral administration and is surrounded by 3, a delayed release layer (optional) 2, a flavor/color/change/texture, hot or cold/sound signalling layer, and 1, an outer coating for intraoral administration.

### Intraoral/Swallow Formulations

(1) An analgesic formulation containing Butorphenol Tartrate 1-2 mg for intraoral and Rofecoxib 12.5-50mg for oral administrations:
(A) Preparation of Rofecoxib core:

| **INGREDIENTS** | **% Weight** | **Typical Example Mg/tablet** |
|---|---|---|
| Rofecoxib | 5 - 30 | 50 |
| Lactose monohydrate | 35 - 60 | 104 |
| Microcrystalline cellulose | 15 - 40 | 50 |
| Starch, pregelatinized | 5 - 20 | 20 |
| Magnesium stearate | 0.1 - 2.0 | 1 |
| **Sub Total** | | 225 |

Procedure:
1. Prepare a granulation containing Rofecoxib, lactose monohydrate, microcrystalline cellulose and starch. Blend with magnesium stearate for 5 - 10 minutes.
2. Compress tablets of 225 mg with a suitable tooling.

(B) Preparation of delayed release Rofecoxib core:

| **INGREDIENTS** | **% Weight** | **Typical Example Mg/tablet** |
|---|---|---|
| Eudragit S100 | 2 - 8 | 10.6 |
| Triethyl citrate | 1 - 6 | 5.3 |
| Talc | 0. 5 - 3 | 2.6 |
| Ammonium (from 1N solution) | 0.04 - 0.4 | 0.19 |
| Water | (To be evaporated) | - |
| Rofecoxib core (A) | 85 - 97 | 225 |
| **Sub Total** | | 243.69 |

Procedure:
1. Prepare the coating solution by mixing water, Eudragit S100, ammonium solution, triethyl citrate and talc to form a uniform dispersion.
2. Coat Rofecoxib core (from (A)) with Eudragit S coating solution using a coating pan or a fluid bed coater until a desired coat weight is achieved.

(C) Preparation of outer layer coating for Butorphanol - The finished product.

| **INGREDIENTS** | **% Weight** | **Typical Example Mg/tablet** |
|---|---|---|
| Butorphanol tartrate | 0.2 - 5 | 2 |
| Opadry ¹ | 3 - 10 | 10 |
| (Optional citric acid/sodium citrate) | 1 - 5 | 5 |
| Aspartame | 0.02 - 2 | 0.3 |
| Flavoring agent | 0.3 - 5 | 2 |
| Water | (To be evaporated) | - |
| Rofecoxib Delayed Release Core (B) | 85 - 97 | 243.69 |
| Total (Finished dosage form) | | 262.09 |
| Key: 1, Product of Colorcon. It contains hydroxypropyl methylcellulose, polyethylene glycol, and other colorants. | | |

Procedure:
1. Prepare a coating solution containing Butorphanol tartrate, Opadry, Aspartame, and the flavoring agent. Citric acid and sodium citrate are optional for a tartness taste.
2. Coat Rofecoxib delayed release core (from (B)) with the coating solution containing Butorphanol tartrate using a coating pan or a fluid bed coater until a desired coat weight is achieved.

(D) Description of the analgesic formulation design:
The product contains 2 mg Butorphanol tartrate in the outer coating layer for intraoral release and 50 mg of Rofecoxib in the delayed release core which starts to release Rofecoxib 2 to 4 hours after administration of the dosage form.
The flavoring agent in the outer layer acts as a flavoring agent for intraoral administration as well as an indicator to the patient of the substantial completion of intraoral administration and the beginning of oral administration. After the patient detects the signalling system (the release of the flavoring from the outer coating layer and/or the sensation of a smooth surface of the delayed release core) he is informed that the outer coating has been substantially dissolved and is instructed to swallow the remaining core for further release of the orally administered dosage form.
The flavoring agent in the outer layer acts as a flavoring agent for intraoral administration. Aspartame is a sweetening agent to reduce bitterness of Butorphanol.
The dosage preparation provides two consecutive doses of analgesic agents. Butorphanol and Rofecoxib. Butorphanol is released from the outer layer intraorally to provide a rapid onset and to avoid first pass metabolism. Rofecoxib is released 2 to 4 hours later to provide a continuous analgesic activity for additional approximately 24 hours.
(2) A hypnotic formulation containing Zolpidem tartrate 5-10 mg for intraoral administration and 5-10 mg for oral administrations:
(A) Preparation of Zolpidem tartrate core:

| **INGREDIENTS** | **% Weight** | **Typical Example Mg/tablet** |
|---|---|---|
| Zolpidem tartrate | 2 - 10 | 5 |
| Lactose monohydrate | 50 - 75 | 98.2 |
| Microcrystalline cellulose | 15 - 40 | 40 |
| Starch, pregelatinized | 5 - 20 | 16 |
| Water | (To be evaporated) | - |
| Magnesium stearate | 0.1 - 2.0 | 0.8 |
| **Sub Total** | | 160 |

Procedure:
1. Prepare a granulation containing Zolpidem tartrate, lactose monohydrate, Microcrystalline cellulose and Starch. Blend with magnesium stearate for 5 - 10 minutes.
2. Compress tablets of 160 mg with a suitable tooling.

(B) Preparation of delayed release Zolpidem tartrate core:

| **INGREDIENTS** | **% Weight** | **Typical Example Mg/tablet** |
|---|---|---|
| Eudragit L 100 | 3 - 8 | 8 |
| Triethyl citrate | 0.3 - 2 | 0.8 |
| Talc | 0.5 - 3 | 2 |
| Polyethylene glycol 6000 | 0.2 - 2 | 0.65 |
| Sodium hydroxide | 0.05 - 0.2 | 0.13 |
| Water | (To be evaporated) | - |
| Zolpidem tartrate core (A) | 85 - 98 | 160 |
| **Sub Total** | | 171.58 |

Procedure:
1. Prepare the coating solution by mixing water, Eudragit L 100, Sodium hydroxide, PEG 6000, triethyl citrate and talc to form a uniform dispersion.
2. Coat Zolpidem tartrate core (from (A)) with Eudragit L coating solution using a coating pan or a fluid bed coater until a desired coat weight is achieved.

(C) Preparation of the signalling system- a Flavoring layer on top of the delayed release Zolpidem core:

| **INGREDIENTS** | **% Weight** | **Typical Example Mg/tablet** |
|---|---|---|
| Opadry¹ | 2 - 10 | 10 |
| Flavoring agent 1 | 0.1 - 2 | 0.5 |
| Tartaric acid | 0.1 - 2 | 0.5 |
| Water | (To be evaporated) | - |
| Zolpidem tartrate delayed release core (B) | 85 - 98 | 171.58 |
| **Sub Total** | | 182.58 |

Key: 1, Product of Colorcon. It contains hydroxypropyl methylcellulose, polyethylene glycol, and other colorants.
Procedure:
1. Prepare the coating solution by mixing water, Opadry, tartaric acid and flavoring agent 1 to form a uniform dispersion.
2. Coat Zolpidem tartrate delayed release core (from (B) ) with the above flavoring coating solution using a coating pan or a fluid bed coater until a desired coat weight is achieved.

(D) Preparation of outer layer coating for Zolpidem - The finished product

| **INGREDIENTS** | **% Weight** | **Typical Example Mg/tablet** |
|---|---|---|
| Zolpidem tartrate | 1 - 5 | 5 |
| Opadry¹ | 3 - 10 | 10 |
| Aspartame | 0.05 - 2 | 0.3 |
| Flavoring agent 2 | 0.05 - 5 | 0.5 |
| Water | (To be evaporated) | - |
| Zolpidem tartrate flavored delayed release core (C) | 85 - 97 | 182.58 |
| **Total (Finished Product)** | | 198.38 |

Key: 1, Product of Colorcon. It contains hydroxypropyl methylcellulose, polyethylene glycol, and other colorants. Flavoring Agent 2 is different from Flavoring Agent 1. Procedure:
1. Prepare a coating solution containing water, Zolpidem tartrate, Opadry, Aspartame and flavoring agent 2.
2. Coat Zolpidem flavored delayed release core (from (C)) with the coating solution containing Zolpidem tartrate using a coating pan or a fluid bed coater until a desired coat weight is achieved.

(E) Description of the Hypnotic formulation design:
The product contains 5mg Zolpidem tartrate in the outer coating layer for intraoral release and 5 mg of Zolpidem tartrate in the delayed release core for Zolpidem tartrate release 0.5 to 2 hours after administration of the dosage form.
The flavoring agent layer between the delayed release core and the outer drug layer acts as an indicator to the patient of the substantial completion of the intraoral administration and the beginning of the oral administration. After the patient detects the signaling system (tart taste and flavoring agent 1 and/or the smooth texture of the surface of inner core) he is informed that the outer coating has substantially dissolved and is instructed to swallow the remaining core for further release of the orally administered dosage form.
The flavoring agent in the outer layer acts as a flavoring agent for intraoral administration. Aspartame is a sweetening agent to reduce bitterness of Zolpidem.
The dosage preparation provides two consecutive doses of a hypnotic agent. The first dose of Zolpidem is released from the outer layer intraorally to provide a rapid onset and to avoid first metabolism and a second dose is released 0.5 to 2 hours later to provide a continuous hypnotic activity for a total of approximately 6 to 10 hours.
(3) A migraine formulation containing Ergotamine Tartrate 2 mg for intraoral administration and Caffeine 100 mg for oral administration:
(A) Preparation of Caffeine granulation:

| **INGREDIENTS** | **% Weight** | **Typical Example Mg/tablet** |
|---|---|---|
| Caffeine | 30 - 70 | 100 |
| Lactose monohydrate | 15 - 40 | 48 |
| Microcrystalline cellulose | 10 - 30 | 40 |
| Povidone | 2 - 8 | 10 |
| Magnesium stearate | 0.1 - 2.0 | 1.0 |
| Stearic acid | 0.1 - 2 | 1.0 |
| **Sub Total** | | 200 |

Procedure:
1. Prepare a granulation containing Caffeine, lactose monohydrate and povidone. Blend the granulation with microcrystalline cellulose and then blend with magnesium stearate and stearic acid for 5 - 10 minutes.
2. Compress tablets of 200 mg with suitable tooling.

(B) Preparation of Signalling system - a Flavoring layer on top of the caffeine core:

| **INGREDIENTS** | **% Weight** | **Typical Example Mg/tablet** |
|---|---|---|
| Opadry¹ | 2 - 10 | 8 |
| Flavoring agent 1 | 0.1 - 2 | 1 |
| Tartaric acid | 0.1 - 2 | 0.5 |
| Water | (To be evaporated) | - |
| Caffeine core (A) | 85 - 98 | 200 |
| **Sub Total** | | 209.5 |

Key: 1, Product of Colorcon. It contains hydroxypropyl methylcellulose, polyethylene glycol, and other colorants.
Procedure:
1. Prepare the coating solution by mixing water, Opadry, tartaric acid and flavoring agent 1 to form a uniform dispersion.
2. Coat caffeine core (from (A) ) with the above flavoring coating solution using a coating pan or a fluid bed coater until a desired coat weight is achieved.

(C) Preparation of outer layer coating of Ergotamine Tartrate - The finished dosage form

| **INGREDIENTS** | **% Weight** | **Typical Example Mg/tablet** |
|---|---|---|
| Ergotamine Tartrate | 0. 2 - 2.0 | 1 |
| Opadry¹ | 2 - 10 | 10 |
| Aspartame | 0.05 - 2 | 0.3 |
| Flavoring agent 2 | 0.05 - 2 | 0.5 |
| Flavored Caffeine core (B) | 90 - 97 | 209.5 |
| **Total (Finished dosage form)** | | 221.3 |

Key: 1, Product of Colorcon. It contains hydroxypropyl methylcellulose, polyethylene glycol, and other colorants.
Procedure:
1. Prepare a coating solution containing water, Ergotamine Tartrate, Opadry, flavoring agent 2 and Aspartame.
2. Coat Caffeine cores (from B) with coating solution containing Ergotamine tartrate using a coating pan or a fluid bed coater until a desired coat weight is achieved.

(D) Description of the migraine formulation design:
The product contains 1 mg of Ergotamine Tartrate in the outer coating layer for intraoral release and an oral administered dosage of 100 mg of Caffeine that releases within one hour after oral ingestion.
The flavoring agent layer between the core and the outer drug layer acts as an indicator to the patient of the substantial completion of the intraoral administration and the beginning of the oral administration. After the patient detects the signalling system (tart taste and flavoring agent 1 and/or the smooth texture of the surface of inner core) he is informed that the outer coating has substantially dissolved and is instructed to swallow the remaining core for further release of the orally administered dosage form.
The flavoring agent 2 in the outer layer acts as a flavoring agent for intraoral administration. Aspartame is a sweetening agent to reduce bitterness of Ergotamine.
The dosage preparation provides administration of Ergotamine intraorally for a rapid onset and to avoid first pass metabolism. The dosage form also provides a second drug, Caffeine which is released after oral administration of the dosage form.
(4) An antihistamine/decongestant formulation containing Chlorpheniramine 4mg for intraoral administration and Pseudoephedrine 120 mg SR/Loratadine 5mg for oral administration:
(A) Preparation of Pseudoephedrine Extended Release Granulation:

| **INGREDIENTS** | **% Weight** | **Typical Example Mg/tablet** |
|---|---|---|
| Pseudoephedrine sulfate | 25 - 50 | 120 |
| Hydroxypropyl Methylcellulose | 30 - 50 | 150 |
| Ethylcellulose | 5 - 20 | 40 |
| Lactose monohydrate | 10 - 25 | 50 |
| Povidone USP | 3 - 10 | 20 |
| Water/Alcohol | (To be evaporated) | (To be evaporated) |
| Magnesium Stearate | 0.3 - 1.5 | 2 |
| **Sub Total** | | 382 |

Procedure:
1. Dissolve Povidone in water or a mixture of alcohol and water.
2. The pseudoephedrine, hydroxypropyl methylcellulose, ethylcellulose, and lactose monohydrate are blended and granulated with above solution.
3. The granulation is screened or milled and dried.
4. The dried granulation is milled and blended with magnesium stearate for 5 - 10 minutes.

(B) Preparation of Loratadine 5mg granulation

| **INGREDIENTS** | **% Weight** | **Typical Example Mg/tablet** |
|---|---|---|
| Loratadine | 2 - 10 | 5 |
| Lactose monohydrate | 50 - 70 | 91.2 |
| Microcrystalline cellulose | 15 - 40 | 38 |
| Starch pregelatinized | 3 - 15 | 15 |
| Magnesium stearate | 0.3 - 1.5 | 0.8 |
| **Sub Total** | | 150 |

Procedure:
1. Prepare a granulation containing Loratadine, lactose monohydrate, microcrystalline cellulose and starch. Blend with magnesium stearate for 5 - 10 minutes.

(C) Compress two-layer cores using a tableting compressor capable of compressing double layer cores. One layer is compressed with the Pseudoephedrine Extended Release granulation (A) at 382 mg and the second layer is compressed with the Loratadine Immediate Release granulation (B) at 150 mg. The double-layer core weighs approximately 532 mg.
(D) Preparation of delayed release Pseudoephedrine/Loratadine cores:

| **INGREDIENTS** | **% Weight** | **Typical Example Mg/tablet** |
|---|---|---|
| Eudragit S 100 | 3 - 8 | 24 |
| Triethyl citrate | 1 - 5 | 12 |
| Talc | 0.5 - 3 | 6 |
| Ammonium (from 1N solution) | 0.03 - 0.3 | 0.42 |
| Water | (To be evaporated) | - |
| Pseudoephedrine/loratadine double-layer core (C) | 85 - 97 | 532 |
| **Sub Total** | | 574.42 |

Procedure:
1. Prepare the coating solution by mixing water, Eudragit S100, ammonium solution, Triethyl citrate and talc to form a uniform dispersion.
2. Coat Pseudoephedrine/Loratadine double-layer core (from (C)) with Eudragit S coating solution using a coating pan or a fluid bed coater until a desired coat weight is achieved.

(E) Preparation of a Signalling Layer on top of the Pseudoephedrine/Loratadine Delayed Release Core:

| **INGREDIENTS** | **% Weight** | **Typical Example Mg/tablet** |
|---|---|---|
| Flavoring agent 1 | 0.05 - 2 | 0.8 |
| Citric acid | 0.05 - 2 | 0.8 |
| Opadry¹ | 2 - 10 . | 20 |
| Water | (To be evaporated) | (To be evaporated) |
| Pseudoephedrine Delayed Extended Release/Loratadine Immediate Release cores (D) | 87 - 98 | 574.42 |
| **Sub Total** | | 596.02 |

Key: 1, Product of Colorcon. It contains hydroxypropyl methylcellulose, polyethylene glycol, and other colorants.
Procedure:
1. Prepare the coating solution by mixing water, Opadry, Citric acid and flavoring agent 1 to form a uniform dispersion.
2. Coat Pseudoephedrine/Loratadine delayed release cores (from D) with signalling layer solution using a coating pan or a fluid bed coater until a desired coat weight is achieved.

(F) Preparation of outer layer coating of Chlorpheniramine : The finished dosage form

| **INGREDIENTS** | **% Weight** | **Typical Example Mg/tablet** |
|---|---|---|
| Chlorpheniramine maleate | 0.3 - 1.2 | 4 |
| Opadry¹ | 2 - 8 | 30 |
| Flavoring agent 2 | 0.05 - 2 | 1 |
| Aspartame | 0.05 - 2 | 0.65 |
| Water | (To be evaporated) | - |
| Pseudoephedrine/Loratadine flavored delayed release cores (E) | 88 - 98 | 596.02 |
| **Sub Total** | | 631.67 |

Key: 1, Product of Colorcon. It contains hydroxypropyl methylcellulose, polyethylene glycol, and other colorants.
Procedure:
1. Prepare a coating solution containing water, Chlorpheniramine, Opadry, flavoring agent 2 and Aspartame.
2. Coat Pseudoephedrine/Loratadine delayed release cores with signalling coat (E) with coating solution containing Chlorpheniramine using a coating pan or a fluid bed coater until a desired coat weight is achieved.

(G) Description of the antihistamine/decongestant formulation design:
This product contains 4 mg of Chlorpheniramine maleate in the outer coating layer for intraoral release and a delayed release core containing 120 mg of Pseudoephedrine in an extended release and Loratadine 5 mg in immediate release for oral administration. The delayed release core releases its contents about 4 to 6 hours after administration of the dosage unit.
The signalling system between the oral ingested delayed release core and the outer layer acts as the indicator to the patient of the substantial completion of the intraoral administration and the beginning of the oral administration. After the patient detects the signalling system (tart sensation and/or flavoring agent 1 and the smooth texture of the surface of the core) he is informed that the outer coating has substantially dissolved and is instructed to swallow the remaining core for further release of the orally administered dosage form.
The flavoring agent in the outer layer acts as a flavoring agent for intraoral administration. Aspartame is a sweetening agent to reduce bitterness of chlorpheniramine.
The dosage preparation provides two consecutive doses of antihistamines, chlorpheniramine and loratadine which release about 4 to 6 hours apart. The dosage unit may be administered in the evening prior to bed time in order for the patient to absorb chlorpheniramine, a sedating antihistamine, which not only provide to the patient antihistamine activity but also help patient sleep during the night. The second dose of antihistamine, loratadine, a non-sedating antihistamine, is released 4 to 6 hours after dosage unit administration and provides a non-sedating antihistamine activity for day time activity. The dosage unit also releases pseudoephedrine in extended release form 4 to 6 hours after administration and provides a decongestant activity for approximately 12 to 16 hours during day time.
(5) An analgesic formulation containing Fentanyl citrate 0.2 mg for intraoral administration and Morphine 60mg SR for oral administration:
(A) Preparation of Morphine sustained release cores:

| **INGREDIENTS** | **% Weight** | **Typical Example Mg/tablet** |
|---|---|---|
| Morphine sulfate | 10 - 25 | 60 |
| Hydroxypropyl Methylcellulose | 40 - 60 | 180 |
| Lactose monohydrate | 20 - 40 | 80 |
| Silicone dioxide | 0.3 - 1.5 | 2 |
| Stearic acid | 0.3 - 1.5 | 2 |
| **Sub Total** | | 324 |

Procedure:
1. Blend Morphine sulfate, hydroxypropyl methylcellulose, and lactose to form a uniform blend. Add silicone dioxide and stearic acid and blend for additional 5 to 10 minutes.
2. Compress Morphine sulfate sustained release cores at about 324 mg using a suitable tooling.

(B) Preparation of delayed release Morphine sulfate cores:

| **INGREDIENTS** | **% Weight** | **Typical Example Mg/tablet** |
|---|---|---|
| Eudragit L 100 | 2 - 8 | 15 |
| Triethyl citrate | 0.2 - 2 | 1.5 |
| Talc | 0.5 - 3 | 3.8 |
| Polyethylene glycol 6000 | 0.2 - 2 | 1.2 |
| Sodium hydroxide | 0.02 - 0.2 | 0.24 |
| Water | (To be evaporated) | - |
| Morphine sulfate sustained release core (A) | 85 - 98 | 324 |
| **Sub Total** | | 345.74 |

Procedure:
1. Prepare the coating solution by mixing water, Eudragit L 100, Sodium hydroxide, PEG 6000, triethyl citrate and talc to form a uniform dispersion.
2. Coat morphine sulfate sustained release core (from (A)) with Eudragit L coating solution using a coating pan or a fluid bed coater until a desired coat weight is achieved.

(C) Preparation of signalling coating on Morphine delayed release cores:

| **INGREDIENTS** | **% Weight** | **Typical Example Mg/tablet** |
|---|---|---|
| Tartaric acid | 0.05 - 1 | 0.5 |
| Flavoring agent 1 | 0.05 - 1 | 0.5 |
| Opadry¹ | 1 - 8 | 10 |
| Water | (To be evaporated) | - |
| Morphine delayed release core (B) | | 345.74 |
| **Sub Total** | | 356.74 |

Key: 1, Product of Colorcon. It contains hydroxypropyl methylcellulose, polyethylene glycol, and other colorants.
Procedure:
1. Prepare the signalling coating solution by mixing water, Opadry, tartaric acid and flavoring agent 1 to form a uniform dispersion.
2. Coat Morphine delayed core (from (B) ) with flavoring coating solution using a coating pan or a fluid bed coater until a desired coat weight is achieved.

(D) Preparation of outer layer coating of Fentanyl :

| **INGREDIENTS** | **% Weight** | **Typical Example Mg/tablet** |
|---|---|---|
| Fentanyl citrate | 0.3 - 1.2 | 0.2 |
| Opadry¹ | 2 - 8 | 10 |
| Flavoring agent 2 | 0.05 - 0.5 | 0.5 |
| Aspartame | 0.05 - 2 | 0.5 |
| Water | (To be evaporated) | (To be evaporated) |
| Morphine sulfate flavored delayed release cores (C) | 90 - 98 | 356.74 |
| **Total (Finished Product)** | | 367.94 |

Key: 1, Product of Colorcon. It contains hydroxypropyl methylcellulose, polyethylene glycol, and other colorants.
Procedure:
1. Prepare a coating solution containing water, Fentanyl citrate, Opadry, flavoring agent 2 and Aspartame.
2. Coat Morphine sulfate delayed release cores with signalling coat (from (C)) with coating solution containing Fentanyl citrate using a coating pan or a fluid bed coater until a desired coat weight is achieved.

(E) Description of Fentanyl citrate/Morphine analgesic formulation design:
This product contains 0.2 mg of Fentanyl citrate in the outer coating layer for intraoral release and an orally administered dosage of 60mg of Sustained Release Morphine that starts to release within 2 hours after oral ingestion.
The signalling system between the oral ingested delayed release core and the outer layer acts as the indicator to the patient of the substantial completion of the intraoral administration and the beginning of the oral administration. After the patient detects the signalling system (tart sensation and/or flavoring agent 1 and the smooth texture of the surface of the core) he is informed that the outer coating has substantially dissolved and is instructed to swallow the remaining core for further release of the orally administered dosage form.
The flavoring agent in the outer layer acts as a flavoring agent for intraoral administration. Aspartame is a sweetening agent to reduce bitterness of Fentanyl
The dosage preparation provides two consecutive doses of analgesic agents, Fentanyl and morphine. Fentanyl is released and absorbed intraorally for a rapid onset and to avoid first pass metabolism. Fentanyl provides a rapid onset of analgesia lasting approximately 2 hours, after which the orally ingested morphine which was formulated with a 2 hour delayed release provides sustained release analgesia for 8 to 12 hours.

## Claims

1. A pharmaceutical composition in unit dosage form for both intraoral and oral administration to a mammalian patient, said unit dosage form configured to be placed within the mouth of said patient, which comprises:
(a) as a first portion, at least one discrete outer layer comprising a therapeutically effective amount of at least one pharmaceutically active ingredient capable of intraoral administration; and
(b) as a second portion located within said first portion, a therapeutically effective amount of at least one pharmaceutically active ingredient capable of oral administration and which is releasable and orally ingestible by the patent after the outer layer has disintegrated or has dissolved intraorally.

2. The pharmaceutical composition defined in claim 1 in the form of a tablet or capsule.

3. The pharmaceutical composition defined in claim 2 wherein the unit dosage form is a tablet and the second portion of the composition is an inner core or at least one layer of a multi-layer tablet, and the first portion is either an outer coating applied on the core or is one or more of the outer layers of a multi-layer tablet.

4. The pharmaceutical composition defined in claim 2 wherein the unit dosage form is a capsule and the second portion of the composition is an uncoated capsule including the pharmaceutically active ingredient capable of oral administration on which the first portion is applied as an outer layer forming an outer coating.

5. The pharmaceutical composition defined in claim 3 wherein the outer coating is a film coat that is applied as a layer to the inner core.

6. The pharmaceutical composition defined in claim 3 wherein the outer coating is a compression coat that is compressed around the inner core.

7. The pharmaceutical composition defined in claim 5 wherein the film coat comprises the at least one pharmaceutically active ingredient capable of intraoral administration and at least one pharmaceutically acceptable coating polymer selected from the group consisting of cellulose, hydroxypropyl methylcellulose, methyl cellulose, polyvinylpyrrolidone, and polyethylene glycol, a pharmaceutically acceptable plasticizer, a pharmaceutically acceptable glidant and a pharmaceutically acceptable colorant.

8. The pharmaceutical composition defined in claim 6 wherein the compression coat comprises the at least one pharmaceutically active ingredient capable of intraoral administration and at least one pharmaceutically acceptable excipient for intraoral administration of the pharmaceutically active ingredient.

9. The pharmaceutical composition defined in claim 6 wherein the compression coat comprises the at least one pharmaceutically active ingredient capable of intraoral administration formulated in a pharmaceutically acceptable effervescent agent which generates effervescence when contacted with salivary fluid.

10. The pharmaceutical composition defined in claim 3 wherein the first portion comprises one or two outer layers each comprising a therapeutically effective amount of a least one pharmaceutically active ingredient capable of intraoral administration and one or more pharmaceutically acceptable excipients for intraoral administration of the pharmaceutically active ingredient capable of intraoral administration.

11. The pharmaceutical composition defined in claim 3 wherein the outer layer of the multi-layer tablet is formulated with a pharmaceutically acceptable effervescent agent which generates effervescence when contacted with salivary fluid.

12. The pharmaceutical composition defined in claim 7 wherein the film coat further comprises a pharmaceutically acceptable flavoring agent.

13. The pharmaceutical composition defined in claim 3 wherein the inner core is an immediate drug release tablet comprising the pharmaceutically active ingredient capable of oral administration and at least one pharmaceutically acceptable excipient for oral administration of the pharmaceutically active ingredient capable of oral administration.

14. The pharmaceutical composition defined in claim 3 wherein the inner core is in a configuration which provides sustained release of the pharmaceutically active ingredient capable of oral administration and which further provides an immediate drug release layer tablet comprising the pharmaceutically active ingredient capable of oral administration and at least one pharmaceutically acceptable excipient for oral administration of the pharmaceutically active ingredient capable of oral administration.

15. The pharmaceutical composition defined in claim 3 wherein the second portion is the at least one layer of the multi-layer tablet comprising the pharmaceutically active ingredient capable of oral administration and which is an immediate drug release layer.

16. The pharmaceutical composition defined in claim 3 wherein the second portion is the at least one inner layer and provides sustained release of the pharmaceutically active ingredient suitable for oral administration over a period of 0.5 to 24 hours.

17. The pharmaceutical composition defined in claim 3 wherein a delayed release coating covers the inner core and comprises the second portion of the composition and then the first portion of the composition is an outer layer over the delayed release coating to delay release of the pharmaceutically active ingredient capable of oral administration for a period of 0.5 to 12 hours.

18. The pharmaceutical composition defined in claim 17 wherein the delayed release coating comprises one or more pharmaceutically acceptable polymer selected from the group consisting of methyl cellulose, hydroxypropyl cellulose, hydroxyethyl cellulose, hydroxymethyl cellulose, hydroxypropyl methyl cellulose acetate succinate, ethyl cellulose, cellulose acetate phthalate, hydroxypropyl methylcellulose phthalate, cellulose acetate trimellitate, carboxymethylcellulose sodium, acrylic acid polymers and copolymers, polymers or copolymers of methacrylic acid, methyl acrylate, ethyl acrylate, methyl methacrylate, ethyl methacrylate, vinyl acetate, vinyl acetate phthalate, or an azo compound, polyvinyl pyrrolidone, pectin, amylose, shellac, zein, and guar gum.

19. The pharmaceutical composition defined in claim 3 wherein the inner core or a layer of the multi-layer tablet core is chewable and comprises at least one pharmaceutically acceptable excipient suitable for a chewable medication and a flavoring agent.

20. The pharmaceutical composition defined in claim 4 wherein the film coat comprises the pharmaceutically active ingredient capable of intraoral administration and at least one pharmaceutically acceptable coating polymer selected from the group consisting of cellulose, hydroxypropyl methylcellulose, methyl cellulose, polyvinylpyrrolidone, and polyethylene glycol, a pharmaceutically acceptable plasticizer, a pharmaceutically acceptable glidant, a pharmaceutically acceptable colorant, and optionally a pharmaceutically acceptable flavoring agent.

21. The pharmaceutical composition defined in claim 4 wherein the second portion of the composition is a capsule containing the pharmaceutically active ingredient capable of oral administration and a pharmaceutically acceptable excipient for sustained release of the pharmaceutically active ingredient suitable for oral administration to provide a sustained release effect of the pharmaceutically active ingredient for 0.5 to 24 hours.

22. The pharmaceutical composition defined in claim 1 wherein the outer layer disintegrates or dissolves within 10 minutes permitting release of the pharmaceutically active ingredient capable of intraoral administration, when the composition is contacted with saliva during intraoral administration.

23. The pharmaceutical composition defined in claim 22 wherein the second part of the composition containing the pharmaceutically active ingredient capable of oral administration remains intact until the intraoral administration of the pharmaceutically active ingredient capable of intraoral administration has been completed.

24. The pharmaceutical composition defined in claim 22 wherein the outer layer disintegrates immediately to allow a rapid intraoral mucosal absorption of the pharmaceutically active ingredient capable of intraoral administration released from the outer layer.

25. The pharmaceutical composition defined in claim 1 which further comprises a pharmaceutically acceptable signalling system located between the first portion and second portion of the composition, within the first portion of the composition or within the second portion of the composition and that is detectable by the patient upon substantial release of the pharmaceutically active ingredient capable of intraoral administration during intraoral administration thereby informing the patient that it is time to orally ingest the remaining second part of the composition containing the pharmaceutically active ingredient capable of oral administration.

26. The pharmaceutical composition defined in claim 1 wherein the pharmaceutically active ingredient capable of intraoral administration has a first pass metabolism which is avoided by intraoral administration.

27. The pharmaceutical composition defined in claim 1 wherein the pharmaceutically active ingredient capable of intraoral administration has a rapid onset of desired therapeutic effect through intraoral absorption.

28. The pharmaceutical composition defined in claim 1 wherein the pharmaceutically active ingredient capable of intraoral administration is selected from the group consisting of analgesics, antihistamines, antidiarrheals, anxiolytics, hypnotics, stimulants, cardiovascular drugs, pulmonary drugs, anti-hypertensives, anti-emetics, anti-inflammatory drugs, renal drugs, steroids, drugs for neurological disorders, anti-psychotic drugs, drugs for treating endocrine disorders, drugs for promoting immunology, drugs for treating osteoarthritis, drugs for treating glaucoma, drugs for treating allergic rhinitis, drugs for treating anemias and other hematological disorders, drugs for treating infectious diseases, drugs for treatment and symptoms of cancer, drugs for insomnia, and antidiabetic drugs.

29. Use of a pharmaceutical composition for the preparation of a medicament for mammals in unit dosage form as a tablet or capsule for both intraoral and oral administration, said pharmaceutical composition placed within the mouth of a patient, which comprises
(a) as a first portion, at least one discrete outer layer comprising a therapeutically effective amount of at least one pharmaceutically active ingredient capable of intraoral administration; and
(b) as a second portion located within said first portion, a therapeutically effective amount of at least one pharmaceutically active ingredient capable of oral administration and which is releasable and orally ingestible by the patient after the at least one outer layer has disintegrated or has dissolved within the patient's mouth which comprises the steps of:
(i) providing the second portion as an inner tablet core or as at least one layer of a multi-layer tablet core or as an uncoated capsule;
and
(ii) applying the first portion as an outer layer or as several outer layers forming an outer coating on the first portion.

30. Use of a pharmaceutical composition for the preparation of a medicament for mammals in unit dosage form as a tablet or capsule for both intraoral and oral administration, which comprises:
(a) as a first portion, a discrete outer layer comprising a therapeutically effective amount of at least one pharmaceutically active ingredient capable of intraoral administration which provides a rapid onset of a desired therapeutic effect; and
(b) as a second portion located within said first portion, a therapeutically effective amount of at least one pharmaceutically active ingredient capable of oral administration and which is releasable and orally ingestible by a patient after the outer layer has disintegrated or has dissolved under the patient's tongue or elsewhere within the patient's mouth, which comprises the steps of:
(i) placing the pharmaceutical composition under the tongue, against the inner wall of the cheek or the upper or lower vestibular layer or elsewhere within the mouth of said patient;
(ii) retaining the pharmaceutical composition under the tongue or against the inner wall of the cheek or the upper or lower vestibular area or elsewhere within the mouth of the patient until the first portion of the pharmaceutical composition containing pharmaceutically active ingredient capable of intraoral administration has dissolved or has disintegrated thereby substantially releasing the pharmaceutically active ingredient capable of intraoral administration; and
(iii) following step (ii) swallowing whole or chewing and swallowing the second portion of the pharmaceutical composition.

31. The use of a pharmaceutical composition defined in claim 30 wherein the pharmaceutical composition further comprises a pharmaceutically acceptable signalling system located between the first portion and second portion of the composition, within the first portion of the composition or within the second portion of the composition and which following step (ii) further comprises the step of relating a signal from the signalling system to indicate to the patient substantial release of the pharmaceutically active ingredient capable of intraoral administration during intraoral administration in step (ii) thereby informing the patient that it is time to orally ingest the remaining second part of the composition containing the pharmaceutically active ingredient capable of oral administration according to step (iii).
